# EUROPEAN PATENT APPLICATION

(11) **EP 3 662 938 A1**
(43) Date of publication of application: **10.06.2020**
(21) Application number: 18841574.9
(22) Date of filing: 06.07.2018
(51) Int. Cl.: A61L 27/06, A61F 2/28

(54) **THREE-DIMENSIONAL STRUCTURE WITH BIOLOGICAL ACTIVITY AND PRODUCTION METHOD THEREFOR**

(30) Priority: 01.08.2017 JP 2017148853
(71) Applicant: Chubu University Educational Foundation, Kasugai-shi Aichi 487-8501 (JP); Ishihara Sangyo Kaisha, Ltd., Osaka-shi, Osaka 550-0002 (JP)
(72) Inventor: YAMAGUCHI, Seiji, Kasugai-shi Aichi 487-8501 (JP); KAJI, Seiji, Osaka-shi Osaka 550-0002 (JP)
(74) Representative: dompatent von Kreisler Selting Werner - Partnerschaft von Patent- und Rechtsanwälten mbB
(86) International application number: PCT/JP2018/025632
(87) International publication number: WO 2019/026540

(57) **Abstract**

The present invention addresses a problem of providing a three-dimensional structure having bioactivity in which a coating film includes a titanium alkoxide hydrolysis product is coated with high adhesion strength on the surface of a three-dimensional structure main body having a concave section and/or a convex section on the surface, and also providing a method for producing such three-dimensional structure. The three-dimensional structure having bioactivity of the present invention includes a three-dimensional structure main body made of a polymer and having a concave section and/or a convex section on a surface, and having a coating film on the surface of the three-dimensional structure main body, and the coating film that includes a titanium alkoxide hydrolysis product, and the coating film has a thickness of 10 nm to 200 nm. No cracks or peelings of the coating film can be recognized when the concave section and/or the convex section on the surface of the three-dimensional structure is observed with a scanning electron microscope at a magnification of 300, and the coating film has bioactivity when evaluated under conditions specified in ISO 23317.

## Description

### [Technical Field]

The present invention relates to a three-dimensional structure having bioactivity used as a bone repair material, a joint prosthetic material, an interbody cage, and the like, and a production method therefor.

### [Background Art]

Titanium metal and alloys thereof are mainly used as materials for three-dimensional structures used for bone repair in a portion where a large load is applied in vivo. While these metal and alloys have the advantage of good bone-bonding ability, the problem thereof is that since they have a much higher modulus of elasticity than bone, stress shielding (phenomenon that no stress acts on the bone, resulting in decrease of the bone weight) occurs, and also where the three-dimensional structure is an interbody cage or the like, subsidence of the cage or the like occurs.

In order to solve such problems, in recent years, a polymer material having an elastic modulus close to that of bone have been used as a material for bone repair. For example, interbody spacers made of polyetheretherketone (PEEK) have been put into practical use.

Meanwhile, since polymers such as PEEK do not have bone-bonding ability, methods for forming a coating film made of titanium oxide or the like on the surface of a three-dimensional structure main body made of a polymer such as PEEK, thereby imparting bone-bonding ability to the three-dimensional structure main body, have been studied.

For example, Patent Literature 1 to 3 and Non Patent Literature 1 disclose a method for forming a titanium oxide coating film on a three-dimensional structure main body made of a polymer such as PEEK by a general sol-gel method using a titanium alkoxide. Specifically, Patent Literature 1 and Non Patent Literature 1 indicate that the coating film is formed by a treatment (referred to hereinbelow as "dip coating") in which a three-dimensional structure main body is dipped in a sol prepared by partially hydrolyzing titanium tetraisopropoxide (TTIP), pulled out of the sol, and dried.

As another method for forming a coating film on the surface of a three-dimensional structure main body, Patent Literature 4 discloses a method using a so-called spin coating in which a three-dimensional structure main body is fixed to a spin coater, and a titanium oxide sol or the like is dropped and coated on the three-dimensional structure main body, while rotating the main body, followed by drying.

### [Citation List]

### [Patent Literature]

[Patent Literature 1] Japanese Patent Application Publication No. 2015-136553
[Patent Literature 2] Japanese Patent No. 4606165
[Patent Literature 3] Japanese Patent No. 5271907
[Patent Literature 4] Japanese Patent No. 6073293

### [Non Patent Literature]

[Non Patent Literature 1] Takashi Kizuki, et al. Apatite-forming PEEK with TiO2 surface layer coating; J Mater Sci: Mater Med (2015) 26:41

### [Summary of Invention]

### [Technical Problem]

However, the following problems arise when a three-dimensional structure having a coating film is produced using dip coating.

For example, where a dip coating is performed on a three-dimensional structure main body, excellent adhesion can be obtained for a coating film formed on a flat portion where the surface of the three-dimensional structure main body is flat. However, for example, where the three-dimensional structure is an interbody spacer, a concavo-convex shape having a sawtooth-shaped cross section is formed on the spacer surface in order to prevent the interbody spacer from being displaced after being inserted in vivo. Therefore, a three-dimensional structure main body in which a concavo-convex shape having a sawtooth-shaped cross section is formed on the surface needs to be used to constitute the interbody spacer. Where concave sections and convex sections are present on the surface of the three-dimensional structure main body in this way, a sol is likely to be stored at the bottom portions of the concave sections or the base portions of the convex sections when the dip coating is performed and film thickness unevenness occurs in the resulting coating film, As a result, adhesion to the three-dimensional structure main body at a thick film portion becomes insufficient, and cracking or peeling occurs. It is presumed that a large stress generates in the coating film due to the film thickness unevenness of the coating film obtained after drying, and this stress causes fissures and cracks, resulting in peeling in the relatively thick part of the three-dimensional structure main body.

A problem arising when a three-dimensional structure provided with a coating film having a portion with poor adhesion as described above is used as a bone repair material or the like is that the coating film peels off from the three-dimensional structure main body during use in vivo and no integration with a bone can be achieved.

By appropriately setting the conditions of the dip coating, it is possible to reduce the thickness of the coating film, thereby making it possible to suppress the peeling of the coating film, but this approach cannot be said to be practical because as the film thickness decreases, bone-bonding ability also decreases or disappears.

Thus, according to the methods for forming a coating film on a three-dimensional structure main body disclosed in Patent Literature 1 and Non Patent Literature 1, although a useful coating film can be coated when the three-dimensional structure main body has no concave sections and convex sections, it is difficult to say that an interbody spacer or the like having a practical concavo-convex shape can be produced by these methods.

Further, in all of the methods for forming a coating film by dip coating or spin coating disclosed in Patent Literature 2 to 4, a coating film is formed on three-dimensional structure main bodies configured of a flat portion having no concave sections or convex sections, and when a coating film is formed by these methods on a three-dimensional structure main body having concave sections and/or convex sections, it is unclear whether the obtained three-dimensional structure is provided with a coating film having high utility.

The present invention has been accomplished with the foregoing in view, and it is an object thereof to provide a three-dimensional structure main body having bioactivity in which a coating film includes a titanium alkoxide hydrolysis product is coated with high adhesion strength on the surface of a three-dimensional structure main body having a concave section and/or a convex section on the surface, and also to provide a method for producing such three-dimensional structure.

### [Solution to Problem]

As a result of intensive studies, the present inventors have found that by applying a centrifugal force to a three-dimensional structure main body having a concave section and/or a convex section on the surface in a state in which a coating film precursor dispersion liquid comprises a coating film precursor includes a titanium alkoxide partial hydrolysis product is coated on the surface of the three-dimensional structure main body, the excess coating film precursor dispersion liquid stored at the bottom portion of the concave section or the base portion of the convex section is spun off or cast, and therefore the thickness of the coating film produced by the titanium alkoxide hydrolysis product on any surface of the concave section, the convex section and the flat section can be made uniform, and as a result, the obtained coating film has high adhesion strength. The present invention has been accomplished based on this finding.

The three-dimensional structure having bioactivity of the present invention has a three-dimensional structure main body made of a polymer and having a concave section and/or a convex section on a surface, and having a coating film on the surface includes the concave section and/or convex section of the three-dimensional structure main body, and the coating film that includes a titanium alkoxide hydrolysis product, and the coating film has a thickness of 10 nm to 200 nm, wherein
no cracks or peelings of the coating film can be recognized when the concave section and/or convex section on the surface of the three-dimensional structure is observed with a scanning electron microscope at a magnification of 300; and
the coating film has bioactivity when evaluated under conditions specified in ISO 23317.

In the three-dimensional structure having bioactivity of the present invention, the coating film preferably has an adhesion strength measured by a 180 degree peeling test in accordance with JIS K 6854 of 40 N/10 mm or higher.

In the three-dimensional structure having bioactivity of the present invention, the coating film preferably has a thickness of 20 nm to 200 nm.

In the three-dimensional structure having bioactivity of the present invention, the three-dimensional structure main body is preferably made of polyetheretherketone.

The three-dimensional structure having bioactivity of the present invention is preferably used as a bone repair material, a joint prosthetic material, or an interbody cage.

A method for producing a three-dimensional structure having bioactivity of the present invention comprises:
a main body preparation step of preparing a three-dimensional structure main body having a concave section and/or a convex section on a surface;
a dispersion liquid preparation step of preparing a coating film precursor dispersion liquid comprises a coating film precursor includes a titanium alkoxide partial hydrolysis product by mixing water and 0.08 parts by mole to 1.5 parts by mole of a titanium alkoxide with respect to 37 parts by mole of an organic solvent;
a precursor coating step of coating the coating film precursor dispersion liquid on the surface of the three-dimensional structure main body, and a coating film forming step of forming a coating film includes the titanium alkoxide hydrolysis product on the surface of the three-dimensional structure main body by rotating the three-dimensional structure main body so that a centrifugal force acts on a center of gravity thereof; and
a bioactivation treatment step of performing bioactivation treatment of the coating film.

It is preferable that the method for producing a three-dimensional structure having bioactivity of the present invention includes a main body modification treatment step of modifying the surface of the three-dimensional structure main body before performing the precursor coating step.

In the method for producing a three-dimensional structure having bioactivity of the present invention, it is preferable that a relative centrifugal acceleration of the centrifugal force acting on the center of gravity of the three-dimensional structure main body is 10 G to 500 G in the coating film forming step.

In the method for producing a three-dimensional structure having bioactivity of the present invention, it is preferable that the three-dimensional structure main body coated with the coating film by the rotation is subjected to drying at a temperature of 50°C to 200°C in the coating film forming step.

### [Advantageous Effects of Invention]

With the three-dimensional structure having bioactivity of the present invention, the surface of the three-dimensional structure main body having a concave section and/or a convex section on the surface is coated with high adhesion strength with a coating film include a titanium alkoxide hydrolysis product. Since the coating film has bioactivity when evaluated under the conditions specified in ISO 23317, that is, has excellent bone-bonding ability, the three-dimensional structure can be used as a bone repair material, a joint prosthetic material, and an interbody cage in vivo and can withstand long-term use.

### [Brief Description of Drawings]

[Fig. 1] Fig. 1 is a photograph showing an example of an interbody cage, which is a three-dimensional structure having bioactivity of the present invention.
[Fig. 2] Fig. 2 is a schematic view of the three-dimensional structure main body used in Example 1; (a) is a plan view, (b) is a side view, and (c) is an enlarged side view of a portion surrounded by a broken line in (b).
[Fig. 3] Fig. 3 is a schematic plan view showing a state in which the three-dimensional structure main body used in Example 1 is fixed on a rotating substrate.
[Fig. 4] Fig. 4 is a scanning electron micrograph taken at a magnification of 50 on the surface of the coating film produced in Example 1.
[Fig. 5] Fig. 5 is a scanning electron micrograph taken at a magnification of 300 on the surface of the coating film produced in Example 1.
[Fig. 6] Fig. 6 is a scanning electron micrograph taken at a magnification of 1,000 on the surface of the coating film produced in Example 1.
[Fig. 7] Fig. 7 is a scanning electron micrograph taken at a magnification of 1,000 on the surface of the coating film produced in Example 1 after bioactivity evaluation.
[Fig. 8] Fig. 8 is a scanning electron micrograph taken at a magnification of 50 on the surface of the coating film produced in Comparative Example 1.
[Fig. 9] Fig. 9 is a scanning electron micrograph taken at a magnification of 300 on the surface of the coating film produced in Comparative Example 1.
[Fig. 10] Fig. 10 is a scanning electron micrograph taken at a magnification of 1,000 on the surface of the coating film produced in Comparative Example 1.
[Fig. 11] Fig. 11 is a scanning electron micrograph taken at a magnification of 1,000 on the surface of the coating film produced in Comparative Example 5 after bioactivity evaluation.
[Fig. 12] Fig. 12 is a scanning electron micrograph taken at a magnification of 1,000 on the surface of the coating film produced in Comparative Example 6 after bioactivity evaluation.

### [Description of Embodiments]

Hereinafter, embodiments of the present invention will be described in detail.

The three-dimensional structure having bioactivity of the present invention comprises a three-dimensional structure main body made of a polymer and having a concave section and/or a convex section on a surface, and having a coating film on a surface of the three-dimensional structure main body and includes a titanium alkoxide hydrolysis product, and the coating film has a thickness of 10 nm to 200 nm. The three-dimensional structure has the following features (1) and (2) .
(1) When the concave section and/or convex section on the surface of the three-dimensional structure is observed with a scanning electron microscope at a magnification of 300, no cracks or peelings of the coating film can be recognized.
(2) The coating film has bioactivity when evaluated under conditions specified in ISO 23317.

### [Three-Dimensional Structure Main Body]

Any polymer suitable for a bone repair material or the like can be used as the polymer constituting the three-dimensional structure main body. Specific examples include polymers such as polyacrylic acid, polymethacrylic acid and these salts thereof, polyethylene, polypropylene, polytetrafluoroethylene, tetrafluoroethylene - perfluoroalkyl vinyl ether copolymer, tetrafluoroethylene - hexafluoropropylene copolymer, tetrafluoroethylene - ethylene copolymer, polyvinylidene fluoride, polychlorotrifluoroethylene, chlorotrifluoroethylene - ethylene copolymer, polyethylene terephthalate, polyamides, polyurethanes, polysiloxanes, polysiloxane elastomers, polyarylketone resins, polysulfone resins and the like.

The polyarylketone resin is a thermoplastic resin having an aromatic nucleus bond, an ether bond and a ketone bond in a structural unit thereof, and many such resins have a linear polymer structure in which benzene rings are bonded by an ether bond and a ketone bond. Representative examples of the polyarylketone resin include polyetherketone (PEK), polyetheretherketone (PEEK), polyetherketoneketone (PEKK), polyetherketoneetherketoneketone (PEKEKK), and the like. Among these, from the viewpoint of having an elastic modulus close to that of bones, it is preferable that polyetheretherketone (PEEK) is used as the polymer constituting the three-dimensional structure main body.

The polysulfone resin (PSF) is an amorphous thermoplastic resin having a sulfonyl group in a structural unit thereof, and many such resins include an aromatic ring for high functionality. A polyethersulfone resin (PES) and a polyphenylsulfone resin (PPSU) are also included as sulfonyl group-containing resins in the polysulfone resins.

The three-dimensional structure main body has at least one concave section and/or convex section on the surface depending on the use of the finally obtained three-dimensional structure having bioactivity (such as a bone repair material, a joint prosthetic material, and an interbody cage). For example, it may have at least a pair of concavo-convex structures. The three-dimensional structure having a concave section and/or a convex section on the surface thereof is inclusive of a structure in which a cavity connecting with the outside is formed inside the three-dimensional structure main body.

Specifically, the concave section and/or the convex section formed on the surface of the three-dimensional structure main body has, for example, a depth of the concave section or a height of the convex section in the range of 0.2 mm or higher, preferably in the range of 0.2 mm to 20 mm, more preferably in the range of 0.2 mm to 5 mm. Further, a width of the concave section or the convex section is, for example, 2.0 mm to 15 mm.

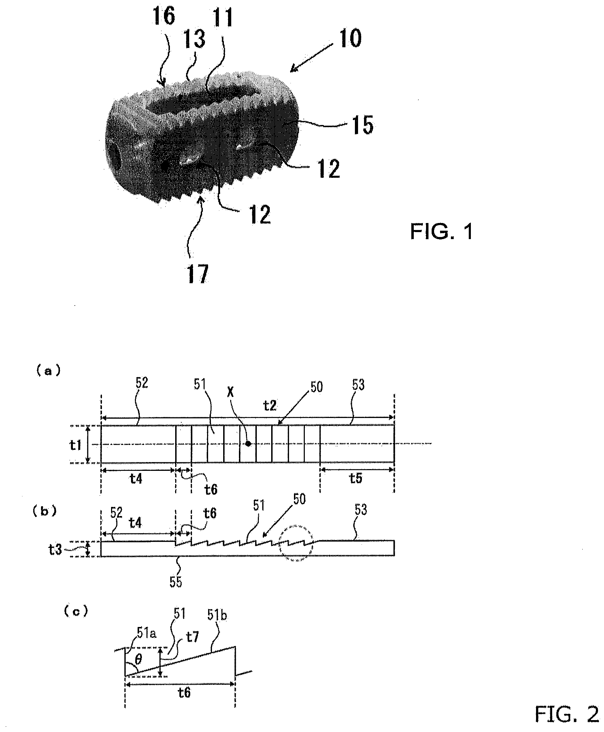

As an example of a three-dimensional structure having a cavity inside, an interbody cage, which is a type of interbody spacer, is shown in Fig. 1.

An interbody cage 10 has a substantially rectangular parallelepiped shape extending in the front-rear direction, and has a through hole 11 having a rectangular cross section and penetrating from an upper surface portion 16 to a lower surface portion 17. A plurality of lateral holes 12 connecting with the through hole 11 is formed in a side surface portion 15 of the interbody cage 10.

Further, a plurality of ridges 13 extending in a direction perpendicular to the side surface portion 15 is formed in parallel to each other on the surfaces of the upper surface portion 16 and the lower surface portion 17 of the interbody cage 10. The plurality of ridges 13 form concave sections and convex sections of the three-dimensional structure main body.

The depth (height) of the concave section and/or convex section in the interbody cage 10 is 0.5 mm, and the width is 5 mm.

The surface on which the coating film in the three-dimensional structure main body is to be formed is preferably subjected to a modification treatment. Specifically, the surface of the three-dimensional structure main body preferably has a water contact angle of 0° to 40°, more preferably 0° to 30°, and still more preferably 0° to 20°.

### [Coating Film]

The coating film formed on the surface of the three-dimensional structure main body is made of a titanium alkoxide hydrolysis product.

Specifically, the titanium alkoxide hydrolysis product constituting the coating film is obtained by drying-induced gelling of a sol formed by partially hydrolyzing and polymerizing a titanium alkoxide. Specifically, when a sol composed of a titanium alkoxide partial hydrolysis product is dried, a titanium alkoxide hydrolysis product is generated by the progress of the hydrolysis reaction and polymerization reaction, and this titanium alkoxide hydrolysis product is called, in general, titanium oxide.

The thickness of the coating film is 10 nm to 200 nm, preferably 20 nm to 200 nm, and more preferably 30 nm to 100 nm.

Where the thickness of the coating film is within the above range, sufficient adhesion of the coating film to the surface of the three-dimensional structure main body is obtained, the coating film is unlikely to peel off even when the three-dimensional structure is used in vivo, and effective bone-bonding ability is obtained for the three-dimensional structure. Meanwhile, where the thickness of the coating film is less than 10 nm, the obtained three-dimensional structure may not have the desired bioactivity (bone-bonding ability). Moreover, where the thickness of the coating film exceeds 200 nm, the coating film is cracked and peeled off, and there is a possibility that the bondability with the bone component (bone-bonding ability) cannot be ensured.

The thickness of the coating film can be measured by observing a cross section using a transmission electron microscope.

As another method for measuring the thickness of the coating film, optical measurement can be performed using an optical interference type film thickness meter (for example, "FE-3000" (manufactured by Otsuka Electronics Co., Ltd.)), an ellipsometer (for example, "UVISEL2" (Horiba, Ltd.)), and the like. Further, the thickness of the coating film can be also measured by a method (calibration curve method) of measuring the optical density of the three-dimensional structure (UV absorptivity by regular transmitted light measured using "UV-2550" (manufactured by Shimadzu Corporation)) and calculating the film thickness by using a calibration curve prepared in advance. In addition, it is also possible to prepare a sample for measuring the thickness of the coating film by coating a dispersion liquid (coating film precursor dispersion liquid) including a titanium alkoxide partial hydrolysis product, which is to be used for coating a three-dimensional structure, on a borosilicate glass substrate and performing the precursor coating step and the coating film forming step, which are described in detail later, under the same conditions as those relating to the actual three-dimensional structure, and then measure the thickness of the coating film by using the sample.

### [(1) Adhesion Strength]

The coating film is such that when the concave section and/or convex section on the surface of the three-dimensional structure is observed with a scanning electron microscope at a magnification of 300, no cracks or peelings of the coating film can be recognized.

Evaluation of the surface of a three-dimensional structure using a scanning electron microscope (hereinafter referred to as "adhesion strength test (C)") is performed by observing at a magnification of 300.

In addition, the coating film is preferably such that when the concave section and/or convex section on the surface of the three-dimensional structure is observed with a scanning electron microscope at a magnification greater than 300, specifically, a magnification of 1,000, no cracks or peelings of the coating film can be recognized.

The position on the surface of the three-dimensional structure, which is observed with a scanning electron microscope, is a flat planar section and a concave section and/or a convex section.

In addition, it is preferable that peeling of the coating film is not visually recognized when performing a test (hereinafter referred to as "adhesion strength test (A)") by affixing a transparent pressure-sensitive adhesive tape (width 25 mm, adhesion sensitivity 4 N/10 mm) specified in JIS K 5600 on the surface of a three-dimensional structure and then peeling off the tape.

Specifically, Cellotape (registered trademark), manufactured by Nichiban Co., Ltd., can be used as the transparent pressure-sensitive adhesive tape used for the adhesion strength test (A).

In the adhesion strength test (A), it is said that no peeling of the coating film is visually recognized in the case where no pieces of the coating film are found to have adhered to the tape when the pressure-sensitive surface of the transparent pressure-sensitive adhesive tape after the adhesion strength test (A) is visually observed.

The position on the surface of the three-dimensional structure to which the transparent pressure-sensitive adhesive tape is affixed may be a flat planar section, or a part of a concave section and/or a convex section.

It is preferable that the adhesion strength of the coating film measured by a 180 degree peeling test in accordance with JIS K 6854 (hereinafter referred to as "adhesion strength test (B)") is 40 N/10 mm or higher, more preferably 41 N/10 mm or higher, and even more preferably 43 N/10 mm or higher.

This adhesion strength test (B) is performed by affixing "Adhesive tape for acrylic foam structure Y-4950 (adhesion strength 34 N/10 mm (T-type peeling force when SUS304 is targeted), 10 mm width)" (manufactured by 3M Co.) to the surface of a three-dimensional structure, pulling the adhesive tape for acrylic foam structure at a speed of 300 mm/min at 180 degrees, and measuring the peel strength.

The position on the surface of the three-dimensional structure to which the adhesive tape for acrylic foam structure is affixed is a flat planar portion.

### [(2) Bioactivity]

The three-dimensional structure exhibits bioactivity when an in vitro evaluation of the apatite-forming ability of an implant material is performed under the conditions specified in ISO 23317.

Specifically, a simulated body fluid (SBF) specified in ISO 23317 is prepared, a sample (three-dimensional structure) is immersed thereinto and placed at 36.5°C for 3 days, and apatite formation on the surface after 3 days is studied by observing with a scanning electron microscope at a magnification of 1,000. The case where the ratio (coverage) of the area where the apatite has precipitated is less than 10% with respect to the surface area of the sample is represented by "-", the case where the ratio is 10% or more and less than 50% is represented by "+", the case where the ratio is 50% or more and less than 90% is represented by "++", the case where the ratio is 90% or more is represented by "+++", and the film with an apatite coverage of 10% or more is evaluated as having bioactivity.

With the three-dimensional structure having bioactivity such as described hereinabove, the surface of the three-dimensional structure main body having a concave section and/or a convex section on the surface is coated with high adhesion strength with a coating film includes a titanium alkoxide hydrolysis product. Since the coating film has bioactivity when evaluated under the conditions specified in ISO 23317, that is, has excellent bone-bonding ability, the three-dimensional structure can be used as a bone repair material, a joint prosthetic material, and an interbody cage in vivo and can withstand long-term use.

### [Method for Producing Three-Dimensional Structure]

A method for producing a three-dimensional structure having bioactivity of the present invention includes a main body preparation step of preparing a three-dimensional structure main body having a concave section and/or a convex section on a surface; a dispersion liquid preparation step of preparing a coating film precursor dispersion liquid including a coating film precursor includes a titanium alkoxide partial hydrolysis product by mixing water and 0.08 parts by mole to 1.5 parts by mole of a titanium alkoxide with respect to 37 parts by mole of an organic solvent; a precursor coating step of coating the coating film precursor dispersion liquid on the surface of the three-dimensional structure main body; a coating film forming step of forming a coating film includes the titanium alkoxide hydrolysis product on the surface of the three-dimensional structure main body by rotating the three-dimensional structure main body so that a centrifugal force acts on a center of gravity thereof; and a bioactivation treatment step of performing bioactivation treatment of the coating film.

In the method for producing a three-dimensional structure having bioactivity of the present invention, it is preferable that the three-dimensional structure main body that has been rotated and coated with the coating film is subjected to drying in the coating film forming step. Further, it is preferable that a main body modification treatment step of modifying the surface of the three-dimensional structure main body is performed before performing the precursor coating step.

### <Main Body Preparation Step>

In the main body preparation step, a three-dimensional structure main body is prepared.

Specifically, the three-dimensional structure main body can be produced by cutting a base material made of a polymer on the basis of a shape corresponding to the application of the three-dimensional structure (bone repair material, joint prosthetic material, interbody cage, and the like). The three-dimensional structure main body can also be produced by a molding method such as an injection molding method. Furthermore, the three-dimensional structure main body can also be produced by a three-dimensional additive processes using a 3D printer or the like.

The produced three-dimensional structure main body is preferably washed with water or alcohol.

### <Main Body Modification Treatment Step>

The main body modification treatment step is performed, as necessary, in consideration of the material of the three-dimensional structure main body and the application of the three-dimensional structure. Specifically, the modification treatment of the surface of the three-dimensional structure main body is performed by imparting a hydrophilic group to the surface of the three-dimensional structure main body made of a polymer. Where a hydrophilic group is imparted to the surface of the three-dimensional structure main body made of a polymer, a coating film includes a titanium alkoxide hydrolysis product can be formed more firmly.

For example, a method of plasma treatment in an oxygen atmosphere or a method of ultraviolet irradiation treatment disclosed in Patent Literature 1 or Non Patent Literature 1 can be used as a specific method of modifying the surface of the three-dimensional structure main body. At this time, the time for performing the plasma treatment in an oxygen atmosphere is preferably 30 sec or longer, and more preferably 5 min or longer. Moreover, the time for performing the ultraviolet irradiation treatment is preferably 5 min or longer, and more preferably 30 min or longer. It is considered that a hydrophilic group such as an oxycarbonyl group (-O-C=O) or a carbonyl group (-C=O) is formed by plasma treatment in an oxygen atmosphere or ultraviolet irradiation treatment. As a modification treatment method other than the above method, for example, a method of forming surface roughness by blasting or acid etching may be used.

### <Dispersion Liquid Preparation Step>

In the dispersion liquid preparation step, water and 0.08 parts by mole to 1.5 parts by mole of titanium alkoxide are mixed with 37 parts by mole of an organic solvent to prepare a coating film precursor dispersion liquid including a coating film precursor includes a titanium alkoxide partial hydrolysis product.

In the dispersion liquid preparation step, titanium alkoxide and water are brought into contact with each other to produce a sol-state titanium alkoxide partial hydrolysis product. The hydrolysis rate of the titanium alkoxide can be set, as appropriate, and is preferably 5% to 70%, and more preferably 10% to 50% in terms of mole. Here, the hydrolysis rate of the titanium alkoxide can be calculated from the ratio between the amount of water to be added and the amount of water to hydrolyze the titanium alkoxide at 100%.

Further, when the titanium alkoxide partial hydrolysis product is generated, it is preferable to perform hydrolysis by mixing titanium alkoxide and water in the presence of an acid such as nitric acid and hydrochloric acid or an alkali such as ammonia.

In the method for producing a three-dimensional structure having bioactivity of the present invention, titanium alkoxide is diluted with an organic solvent such as an alcohol and then hydrolyzed. Specifically, water and 0.08 parts by mole to 1.5 parts by mole, preferably 0.09 parts by mole to 1.0 mol part of titanium alkoxide are mixed with 37 parts by mole of organic solvent to prepare a coating film precursor dispersion liquid comprises a coating film precursor includes a titanium alkoxide partial hydrolysis product. It is conceivable that as a result diluting with an organic solvent and hydrolyzing in this way, the titanium alkoxide partial hydrolysis product in the coating film precursor dispersion liquid has an appropriate concentration, thereby making it possible to form a coating film having excellent adhesion on the surface of the three-dimensional structure main body.

It is preferable that the coating film precursor dispersion liquid is prepared by mixing the titanium alkoxide : water : organic solvent : acid or alkali at a:b:37:0.1 (where a is 1.0 to 1.5 and preferably 1.0, and b is 1.0 to 1.5 and preferably 1.0) in a molar ratio of the amount used of each component, partially hydrolyzing the titanium alkoxide, and if necessary, further diluting with an organic solvent so that the amount of titanium alkoxide used is within the above range with respect to the total amount of organic solvent used.

The viscosity of the resulting coating film precursor dispersion liquid is preferably 0.8 mPa·s to 100 mPa·s as measured at 20°C.

The amount of water used relative to 1 mol part of titanium alkoxide is preferably 0.1 parts by mole to 4 parts by mole, and more preferably 0.5 parts by mole to 3 parts by mole.

In addition, the amount of acid or alkali used is preferably 0.01 parts by mole to 2.0 parts by mole, and more preferably 0.05 parts by mole to 1.0 mol part, relative to 1 mol part of the titanium alkoxide.

Specific examples of the titanium alkoxide include titanium alkoxides based on aliphatic alcohols having 1 to 8 carbon atoms, such as tetraethoxytitanate, tetra(n-propoxy)titanate, tetra(isopropoxy)titanate, tetra(n-butoxy)titanate, tetra(isobutoxy)titanate, and tetra(tert-butoxy)titanate.

Specific examples of the organic solvent include alcohols such as methanol, ethanol, propanol, butanol, ethylene glycol and the like; ethers such as dimethyl ether, methyl tert-butyl ether, methyl propyl ether, diethyl ether, ethyl methyl ether, ethyl tert-butyl ether, dibutyl ether, and the like; and the like.

### <Precursor Coating Step>

In the precursor coating step, the coating film precursor dispersion liquid is coated on the surface of the three-dimensional structure main body.

The method of coating the coating film precursor dispersion liquid on the surface of the three-dimensional structure main body is not particularly limited. For example, a method of immersing the three-dimensional structure main body in the coating film precursor dispersion liquid and then pulling up the main body, a method of spraying the coating film precursor dispersion liquid or coating with a brush or the like on the three-dimensional structure main body, a method of wetting the surface of the three-dimensional structure main body with the coating film precursor dispersion liquid, and the like can be used.

When coating the coating film precursor dispersion liquid on the surface of the three-dimensional structure main body, the three-dimensional structure main body is preferably fixed on an appropriate substrate. As a means for fixing, for example, screwing to the substrate or the like can be used, and it is preferable that fixing is performed, for example, using a fixture such as a long screw to prevent contact with the substrate, so that the coating film precursor dispersion liquid could be coated on the entire surface of the three-dimensional structure main body, according to the shape and structure of the three-dimensional structure main body.

Where the method of immersing the three-dimensional structure main body in the coating film precursor dispersion liquid and then pulling up the main body is used a method of coating the coating film precursor dispersion liquid on the surface of the three-dimensional structure main body, the speed of pulling up the three-dimensional structure main body from the coating film precursor dispersion liquid is set, as appropriate, in consideration of the thickness of the coating film to be formed, the coating film precursor dispersion liquid, and the like, and is, for example, 1 mm/sec to 100 mm/sec.

### <Coating Film Forming Step>

In the coating film forming step, the three-dimensional structure main body coated with the coating film precursor dispersion liquid is rotated so that centrifugal force acts on the center of gravity thereof, so that a coating film includes a titanium alkoxide partial hydrolysis product or a titanium alkoxide hydrolysis product obtained by a hydrolysis reaction and a polymerization reaction advanced by rotation, drying or the like can be formed on the surface of the three-dimensional structure main body. The coating of the coating film precursor dispersion liquid on the surface of the three-dimensional structure main body and the application of centrifugal force to the three-dimensional structure main body may be performed at the same time, but from the viewpoint of enabling the formation of a coating film with high uniformity in thickness, it is preferable to apply a centrifugal force to the three-dimensional structure main body after the coating film precursor dispersion liquid is coated on the surface of the three-dimensional structure main body.

By rotating the three-dimensional structure main body so that a centrifugal force acts on the center of gravity thereof, the excess coating film precursor dispersion liquid is spun off and removed, or the coating film precursor dispersion liquid is cast on the surface of the three-dimensional structure main body to improve thickness uniformity of the film formed by the dispersion liquid.

Examples of the method for rotating the three-dimensional structure main body include methods using a spin coater, a centrifuge, a centrifugal separator and the like, and it is particularly preferable to use a method using a centrifuge or a centrifugal separator.

In the rotation of the three-dimensional structure main body, the three-dimensional structure main body is fixed so that the rotation center (rotation axis) and the center of gravity of the three-dimensional structure main body are spaced apart from each other, and rotated in a autorotating mode. Furthermore, it is more preferable that the three-dimensional structure main body is rotated in a autorotation-revolution mode in which autorotating is performed in a direction opposite to the rotation direction when viewing from one side to the other side along the rotation axis in a state in which the three-dimensional structure main body is fixed so that the rotation center (rotation axis) and the center of gravity of the three-dimensional structure main body are spaced apart from each other.

The distance between the rotation center (rotation axis) and the position of the center of gravity of the three-dimensional structure main body is, for example, preferably 5 mm or longer, and more preferably 10 mm to 1,000 mm.

In the rotation of the three-dimensional structure main body, it is preferable that all of the concave sections and/or convex sections of the three-dimensional structure main body are set apart from the rotation center (rotation axis). Moreover, for example, when the concave sections and/or convex sections of the three-dimensional structure main body are composed of ridges or grooves which extend in one direction, the direction in which the ridges or grooves extend may or may not be the same as the direction in which a centrifugal force acts.

In the case of autorotating the three-dimensional structure main body, as a specific condition, a rotary mechanism for autorotating the three-dimensional structure main body in the direction opposite to the rotation direction is provided. The rotary mechanism may be a small motor or may be a cam mechanism that reverses in the rotation direction. The rotational speed of the autorotating can be appropriately set, and is preferably 100 rpm to 10,000 rpm, more preferably 1,000 rpm to 5,000 rpm.

It is preferable that the time is short that from when the coating film precursor dispersion liquid is coated on the surface of the three-dimensional structure main body to when the three-dimensional structure main body is started rotation. Specifically, this time is preferably within 5 min, and more preferably within 30 sec.

In the rotation of the three-dimensional structure main body, the relative centrifugal acceleration of the centrifugal force acting on the center of gravity of the three-dimensional structure main body is preferably 10 G to 500 G, more preferably 20 G to 250 G, and even more preferably 50 to 200 G.

When the relative centrifugal acceleration of the centrifugal force acting on the center of gravity of the three-dimensional structure main body is within the above range, the surplus coating film precursor dispersion liquid is sufficiently spun off, or the coating film precursor dispersion liquid is cast on the surface of the three-dimensional structure main body to improve thickness uniformity of the film formed by the dispersion liquid. The centrifugal acceleration increases in proportion to the absolute value of the distance from the rotation center and the square of the angular velocity of the rotational motion. That is, the centrifugal acceleration a is expressed by a = rω² (where r is a position vector (radius) (m) from the center of rotation, and ω is an angular velocity (rad/s)). Further, the angular velocity is represented by ω = 2πN/60 (where N is the number of revolutions (rpm)). The relative centrifugal acceleration RCF is obtained from the equation of relative centrifugal acceleration RCF (G) = centrifugal acceleration/earth gravity acceleration.

Therefore, the relative centrifugal acceleration of the centrifugal force acting on the center of gravity of the three-dimensional structure main body can be adjusted by changing either or both of the distance between the rotation axis and the center of gravity of the three-dimensional structure main body and the rotation speed related to the rotation of the three-dimensional structure main body.

In the coating film forming step, the coating film of the alkoxide hydrolysis product can be formed by performing, as necessary, a drying of the coating film precursor in the sol state and/or the alkoxide hydrolysis product in the gel state which is coated on the three-dimensional structure main body.

The drying may be performed at a temperature at which the polymer forming the three-dimensional structure main body is not plasticized, and may be natural drying performed by storing in a room or the like, but is preferably performed at a temperature of, for example, 50°C to 200°C, more preferably at a temperature of 70°C to 140°C. The drying time can be set as appropriate, for example, to about 10 min to 48 h.

### <Bioactivation Treatment Step>

In the bioactivation treatment step, the coating film is bioactivated, and the three-dimensional structure is made bioactive.

The bioactivation treatment is performed to allow the coating film to exhibit bone-bonding ability by apatite formation in vivo, and is preferably an acid treatment disclosed in, for example, Patent Literature 1 and Non Patent Literature 1. In the acid treatment, for example, an aqueous solution including at least one acid selected from hydrochloric acid, nitric acid and sulfuric acid is used. The acid concentration in this aqueous solution is preferably 0.001 mol/L or more and 5 mol/L or less, and more preferably 0.01 mol/L or more and 0.5 mol/L or less. By performing the acid treatment by using an aqueous solution having such an acid concentration, the zeta potential of the coating film can be charged to +3 mV to +20 mV in a relatively short time.

In the bioactivation treatment, after acid treatment, washing and drying may be performed as appropriate. The drying is performed at a temperature at which the polymer is not plasticized. Specifically, the drying is preferably performed at a temperature of 50°C to 200°C, more preferably 70°C to 140°C. The drying time can be set, as appropriate, for example, to about 10 min to 48 h.

### [Application]

The three-dimensional structure having bioactivity of the present invention can be used as a bone repair material for bones, teeth and the like, and specifically, as an artificial bone, a bone defect prosthetic material or a filling material. Also, for example, the three-dimensional structure can be used for vertebroplasty, vertebral formation, femur formation, skull defect repair, and the like, and can be used for various cages such as an interbody cage. The three-dimensional structure also can be used as a joint prosthetic material.

The three-dimensional structure having bioactivity of the present invention may optionally include, according to such applications thereof, a radiopaque substance such as barium sulfate or zirconium oxide, and an antibacterial substance such as silver, copper, antibiotics and the like together with the titanium alkoxide hydrolysis product in the coating film.

According to the method for producing a three-dimensional structure having bioactivity as described above, a centrifugal force is caused to act on the three-dimensional structure main body in a state where a coating film precursor dispersion liquid comprises a coating film precursor includes a titanium alkoxide partial hydrolysis product is formed on the surface of a three-dimensional structure main body having a concave section and/or a convex section on the surface, whereby an excess coating film precursor dispersion liquid accumulated at the bottom portion formed by the concave section or the convex section is spun off. Therefore, it is possible to improve thickness uniformity of the coating film formed by the titanium alkoxide hydrolysis product on any surface of the concave section, the convex section, and the flat section. As a result, a three-dimensional structure having bioactivity in which the coating film is coated with high adhesion strength can be produced.

Embodiments of the present invention are described hereinabove, but the present invention is not limited to these embodiments, and various changes can be added.

### Examples

Hereinafter, specific examples of the present invention will be described, but the present invention is not limited thereto.

### [Example 1: Preparation Example of Three-Dimensional Structure [A]]

### (1) Main body preparation step

A three-dimensional structure main body (hereinafter referred to as "main body [A]") made of polyetheretherketone (PEEK) and having the form shown in Fig. 2 was prepared. The main body [A] is a rectangular plate having a vertical width (t1) of 5 mm, a horizontal width (t2) of 39.35 mm, and a thickness (t3) of 2 mm.

The main body [A] has a concavo-convex section (50) in a central region in the long side direction on one surface thereof. The concavo-convex section portion (50) is formed such that nine wedge-shaped grooves (51) extending in the short side direction are arranged in the long side direction. The width (t6) of each groove (51) is 2.15 mm, and the depth (t7) of each groove (51) is 0.5 mm.

In the cross section of the main body [A] cut in the thickness direction along the long side direction, of the two sides related to the inner surface of the groove (51), one side (51a) extends in the thickness direction of the main body [A], the other side (51b) extends in a direction inclined in the thickness direction of the main body [A], and an angle θ formed by the one side (51a) and the other side (51b) is 76.9°.

Flat sections (52, 53) are respectively formed on both sides of the concavo-convex section (50) in the main body [A]. The widths (t4, t5) of the flat sections (52, 53) in the long side direction of the main body [A] are each 10 mm.

Further, the entire other surface of the main body [A] is a flat section (55).

The main body [A] was washed with ethanol, then washed with pure water, and thereafter dried with a dryer.

### (2) Main body modification treatment step

The washed and dried main body [A] was subjected to plasma treatment (hereinafter referred to as "O₂ plasma treatment") by using a vapor deposition device "IE-5" (manufactured by Eiko Co., Ltd.) under the conditions of an oxygen gas partial pressure of 10 Pa, plasma 0.6 kV-8 mA, an anode-main body [A] distance of 55 mm, and a treatment time of 5 min. The contact angle of water on the surface of the O₂ plasma-treated main body [A] was 20°.

### (3) Dispersion liquid preparation step

A TTIP partial hydrolysis product sol [1] was prepared by preparing a solution A in which 0.01 mol of titanium tetraisopropoxide (TTIP) and 0.185 mol of ethanol were mixed and a solution B in which 0.01 mol of water, 0.185 mol of ethanol and 0.001 mol of nitric acid were mixed, and gradually dropping the solution B into the solution A while stirring the solution A. The molar ratio of components used to form the TTIP partial hydrolysis product sol [1] was TTIP : H₂O : EtOH : HNO₃ = 1 : 1 : 37 : 0.1. The hydrolysis rate of TTIP in the obtained TTIP partial hydrolysis product sol [1] was 25% in terms of mole.

This TTIP partial hydrolysis product sol [1] was diluted with ethanol so that the amount of TTIP used was 0.8 parts by mole with respect to the total amount of 37 parts by mole of ethanol used, thereby preparing the coating film precursor dispersion liquid.

The hydrolysis rate of TTIP in the obtained coating film precursor dispersion liquid was 25% in terms of mole.

### (4) Precursor coating step - (5) Coating film forming step

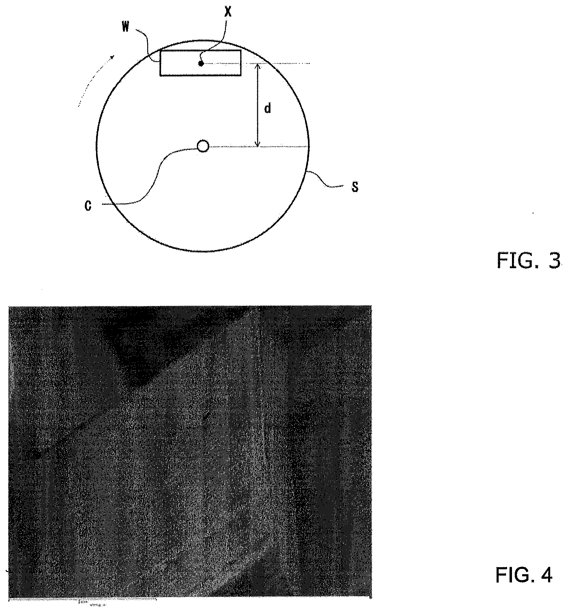

The main body [A] subjected to the O₂ plasma treatment was immersed in the coating film precursor dispersion liquid, submerged at a speed of 10 mm/sec, pulled up at a speed of 10 mm/sec (dip coating). The main body [A] subjected to the dip coating was fixed, as shown in Fig. 3, on a rotating substrate S of a spin coater so that the distance d from the rotation axis C to the center of gravity X of the main body [A] (W in Fig. 3) was 40 mm, and was rotated for 30 sec at a rotation speed of 1,500 rpm. At this time, the relative centrifugal acceleration was set to 100 G.

Next, in a stationary state where no centrifugal force was applied, drying was performed by heating at 80°C for 24 h.

### (6) Bioactivation treatment step

After that, the dried main body [A] was immersed in 0.01 mol/L hydrochloric acid at 80°C for 24 h (hereinafter referred to as "acid treatment"), and washed with pure water for 30 sec, whereby the surface of the main body [A] was subjected to a bioactivation treatment to obtain a three-dimensional structure [A].

### [Examples 2 to 4: Preparation Examples of Three-Dimensional Structures [B] to [D]]

Three-dimensional structures [B] to [D] were obtained in the same manner as in Example 1 except that the coating film precursor dispersion liquids were prepared by diluting the TTIP partial hydrolysis product sol [1] with ethanol so that the amount of TTIP used relative to the total amount of ethanol used in the dispersion liquid preparation step of Example 1 was at ratios shown in Table 1.

### [Comparative Example 1: Preparation Example of Three-Dimensional Structure [E]]

A three-dimensional structure [E] was obtained in the same manner as in Example 1 except that the TTIP partial hydrolysis product sol [1] itself was used as the coating film precursor dispersion liquid in the precursor coating step of Example 1, and the main body [A] subjected to the O₂ plasma treatment immersed therein at a rate of 2 mm/sec, pulled out at a rate of 1 cm/min (dip coating), and then subjected to a drying without rotating.

### [Comparative Examples 2 to 5: Preparation Examples of Three-Dimensional Structure [F] to [I]]

Three-dimensional structures [F] to [I] were obtained in the same manner as in Comparative Example 1 except that the coating film precursor dispersion liquids were prepared by diluting the TTIP partial hydrolysis product sol [1] with ethanol so that the amount of TTIP used relative to the total amount of ethanol used in the dispersion liquid preparation step of Comparative Example 1 was at ratios shown in Table 2.

### [Comparative Example 6: Preparation Example of Three-Dimensional Structure [J]]

A three-dimensional structure [J] was obtained in the same manner as in Example 1 except that the coating film precursor dispersion liquid was prepared by diluting the TTIP partial hydrolysis product sol [1] with ethanol so that the amount of TTIP used relative to the total amount of ethanol used in the dispersion liquid preparation step of Example 1 was at the ratio shown in Table 2.

Using the above three-dimensional structures [A] to [J] as samples, the thickness of the coating film was measured, and the adhesion and bioactivity were evaluated. The results are shown in Tables 1 and 2.

### (1) Measurement of coating film thickness

The thickness of the coating film was measured by a method (calibration curve method) of measuring the optical density (UV absorptivity by regular transmitted light measured using "UV-2550" (manufactured by Shimadzu Corporation)) of the three-dimensional structures [A] to [J] and calculating the film thickness by using a calibration curve prepared in advance.

### (2) Evaluation of adhesion

(A) A transparent pressure-sensitive adhesive tape (width 25 mm, adhesion sensitivity 4 N/10 mm) specified in JIS K 5600 was affixed to the surface of the three-dimensional structure on the flat section of the coating film and peeled off. When the pressure-sensitive adhesive surface of the transparent pressure-sensitive adhesive tape was visually observed, where the coating film pieces were not attached, the adhesion strength was evaluated as high and indicated by "○" in Tables 1 and 2. Where the coating film pieces were attached, the adhesion strength was evaluated as low and indicated by "X" in Tables 1 and 2.
(B) The "adhesive tape for acrylic foam structure Y-4950 (adhesion strength 34 N/10 mm (T-type peeling force when SUS304 is targeted), 10 mm width)" (manufactured by 3M) was affixed to the flat section of the coating film, the adhesive tape for acrylic foam structure was then pulled at a speed of 300 mm/min at 180 degrees, and the peel strength was measured.
   Where the peel strength was 42 [N/10 mm] or higher, the adhesion strength was evaluated as high.
(C) Each of the flat section and the concavo-convex section of the sample was observed at a magnification of 300 by using a scanning electron microscope. The concavo-convex section was also observed with the magnification of 50 and the magnification of 1,000 by using the scanning electron microscope. As a result of observation at a magnification of 300 for the flat section and observation at a magnification of 300 and 1,000 for the concavo-convex section, the adhesion strength was evaluated as high and indicated by "○" in Tables 1 and 2 when no cracking or peeling of the coating film was recognized. Where cracks and peelings were recognized, the adhesion strength was evaluated as low and indicated by "X" in Tables 1 and 2.

### (3) Evaluation of bioactivity

A simulated body fluid (SBF) specified in ISO 23317 was prepared, the sample was immersed thereinto and stored at 36.5°C for 3 days, the formation of apatite on the sample surface was studied after 3 days by thin-film X-ray diffraction, and the amount of apatite formed was studied by observation with a scanning electron microscope at a magnification of 1,000. The case where the ratio (coverage) of the area of the portion where the apatite precipitated was less than 10% with respect to the surface area of the sample was represented by "-", the case where the ratio was 10% or more and less than 50% was represented by "+", the case where the ratio was 50% or more and less than 90% was represented by "++", the case where the ratio was 90% or more was represented by "+++", and the film with an apatite coverage of 10% or more was evaluated as having bioactivity.

**[Table 1]**

| | | Example 1 | Example 2 | Example 3 | Example 4 |
|---|---|---|---|---|---|
| Sample No. | | A | B | C | D |
| TTIP : ethanol | | 0.8 : 37 | 0.4 : 37 | 0.2 : 37 | 0.1 : 37 |
| Film thickness [nm] | | 120 | 68 | 40 | 15 |
| Adhesion (A) | | ○ | ○ | ○ | ○ |
| Adhesion (B) [N/10 mm] | | 42 or higher | 42 or higher | 42 or higher | 42 or higher |
| Cracking, peeling, adhesion (C) | Flat section (300x) | ○ | ○ | ○ | ○ |
| | Concavo-convex section (300x) | ○ | ○ | ○ | ○ |
| | Concavo-convex section (1,000x) | ○ | ○ | ○ | ○ |
| Bioactivity evaluation | | ++ | +++ | ++ | + |

**[Table 2]**

| | | Compar. Example 1 | Compar. Example 2 | Compar. Example 3 | Compar. Example 4 | Compar. Example 5 | Compar. Example 6 |
|---|---|---|---|---|---|---|---|
| Sample No. | | E | F | G | H | I | J |
| TTIP : ethanol | | 1 : 37 | 0.8 : 37 | 0.6 : 37 | 0.4 : 37 | 0.2 : 37 | 0.05 : 37 |
| Film thickness [nm] | | 40 | 25 | 18 | 10 | 4 | 5 |
| Adhesion (A) | | ○ | ○ | ○ | ○ | ○ | ○ |
| Adhesion (B) [N/10 mm] | | 42 or higher | 42 or higher | 42 or higher | 42 or higher | 42 or higher | 42 or higher |
| Cracking, peeling, adhesion (C) | Flat section (300x) | ○ | ○ | ○ | ○ | ○ | ○ |
| | Concavo-convex section (300x) | X | X | X | X | ○ | ○ |
| | Concavo-convex section (1,000x) | X | X | X | X | ○ | ○ |
| Bioactivity evaluation | | +++ | ++ | ++ | + | - | - |

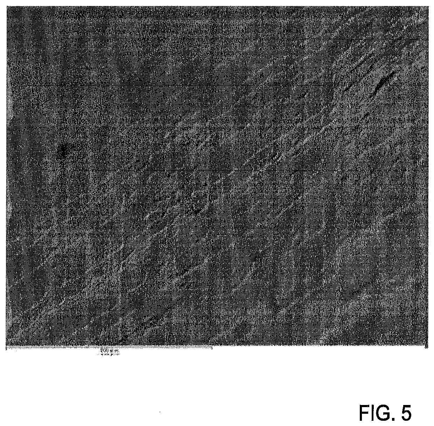

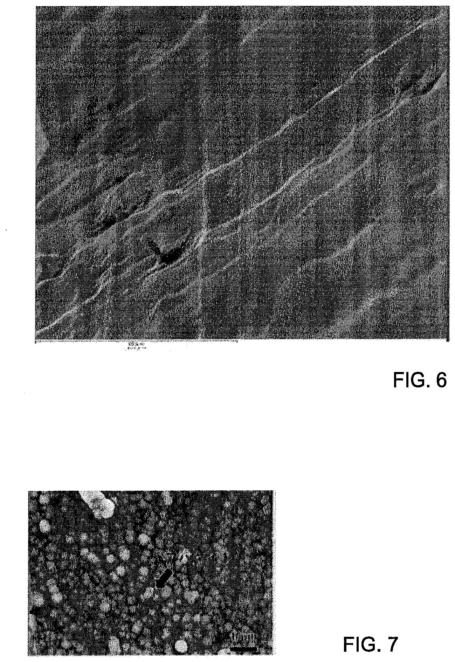

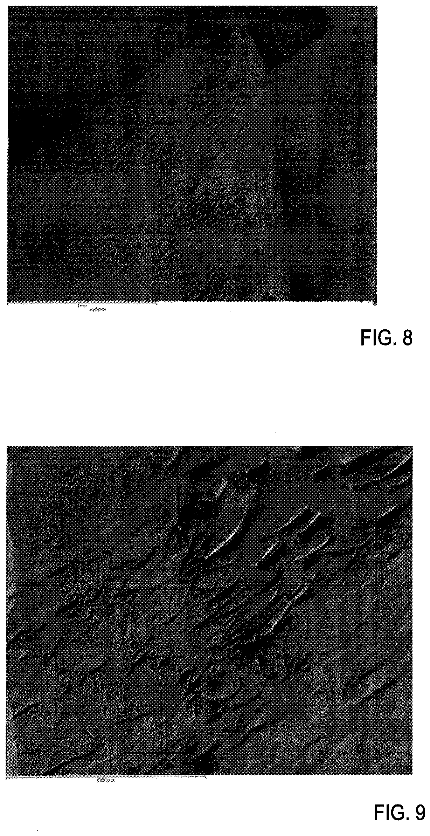

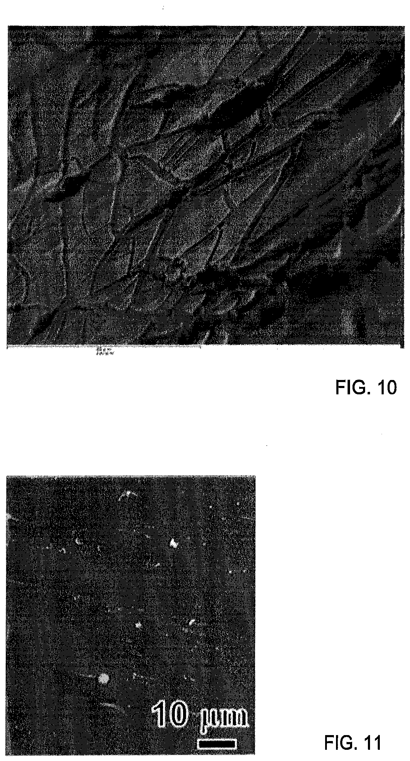

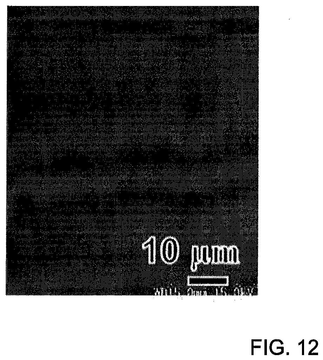

As is apparent from Tables 1 and 2, the three-dimensional structures [A] to [D] according to Examples 1 to 4 all had high adhesion strength, and the coating film did not crack or peeled off and was confirmed to have bioactivity. Meanwhile, the samples of the three-dimensional structures [E] to [H] according to Comparative Examples 1 to 4 in which the centrifugal force was not applied were confirmed to be cracked and peeled off at the concavo-convex section of the three-dimensional structure, and the adhesion strength was confirmed to be low. Fig. 4 shows a scanning electron micrograph of the coating film of the three-dimensional structure [A] according to Example 1 that was captured at a magnification of 50, Fig. 5 shows a scanning electron micrograph of the coating film captured at a magnification of 300, and Fig. 6 shows a scanning electron micrograph of the coating film captured at a magnification of 1,000. Further, Fig. 8 shows a scanning electron micrograph of the coating film of the three-dimensional structure [E] according to Comparative Example 1 that was captured at a magnification of 50, Fig. 9 shows a scanning electron micrograph of the coating film captured at a magnification of 300, and Fig. 10 shows a scanning electron micrograph of the coating film captured at a magnification of 1,000. In addition, for the three-dimensional structures [I] and [J] according to Comparative Example 5 and Comparative Example 6 in which the amount of TTIP used was small relative to the total amount of organic solvent (ethanol) used, a high adhesion strength was obtained because the coating film was thin, but it was confirmed that sufficient bioactivity could not be obtained. Scanning electron micrographs of the surface after evaluating the bioactivity of the three-dimensional structure [A] according to Example 1, the three-dimensional structure [I] according to Comparative Example 5, and the three-dimensional structure [J] according to Comparative Example 6 are shown in Figs. 7, 11, and 12, respectively.

### [Examples 5 to 9: Preparation Examples of Three-Dimensional Structures [K] to [O]]

Three-dimensional structures [K] to [O] were obtained in the same manner as in Example 1 except that in the precursor coating step of Example 1, the main body [A] subjected to the dip coating was fixed to a spin coater so that the distance from the rotation axis C to the center of gravity X of the main body [A] was as shown in Table 3, and the main body was rotated at a rotation speed according to Table 3.

Using the above-mentioned three-dimensional structures [K] to [O] as samples, the thickness of the coating film was measured and the adhesion evaluation (C) was performed in the same manner as for the three-dimensional structure [A]. The results are shown in Table 3.

**[Table 3]**

| | | Example 5 | Example 6 | Example 7 | Example 8 | Example 9 |
|---|---|---|---|---|---|---|
| Sample No. | | K | L | M | N | O |
| Rotation speed [rpm] | | 500 | 1,100 | 1,500 | 3,000 | 1,500 |
| Distance from rotation axis C to center X of gravity of main body [A] [mm] | | 40 | 40 | 40 | 40 | 10 |
| Relative centrifugal acceleration [G] | | 11 | 54 | 101 | 403 | 25 |
| Film thickness [nm] | | 45 | 40 | 38 | 36 | 37 |
| Cracking, peeling, adhesion (C) | Flat section (300x) | ○ | ○ | ○ | ○ | ○ |
| | Concavo-convex section (300x) | ○ | ○ | ○ | ○ | ○ |
| | Concavo-convex section (1,000x) | ○ | ○ | ○ | ○ | ○ |

As is apparent from Table 3, it was confirmed that when the relative centrifugal acceleration was 11 G or higher, high adhesion strength was obtained for the coating film, and neither cracks nor peelings occurred at the concavo-convex section of the three-dimensional structure.

### [Industrial Applicability]

In the three-dimensional structure having bioactivity of the present invention, the surface of the three-dimensional structure main body having a concave section and/or a convex section on the surface is coated with high adhesion strength with a coating film includes a titanium alkoxide hydrolysis product and having high uniformity of thickness. Further, since the coating film has bioactivity when evaluated under the conditions specified in ISO 23317, that is, has excellent bone-bonding ability, the three-dimensional structure can be advantageously used as a bone repair material, a joint prosthetic material, and an interbody cage in vivo.

### [Reference Sings List]

- 10: Interbody cage
- 11: Through hole
- 12: Transverse hole
- 13: Projection
- 15: Side surface portion
- 16: Upper surface portion
- 17: Lower surface portion
- 50: Concavo-convex section
- 51: Groove
- 51a: One side
- 51b: Other side
- 52, 53, 55: Flat sections
- C: Rotation axis
- S: Rotating substrate
- W: Main body [A]
- X: Center of gravity of main body [A]

## Claims

1. A three-dimensional structure having bioactivity, comprising: a three-dimensional structure main body made of a polymer and having a concave section and/or a convex section on a surface, and having a coating film on the surface includes the concave section and/or convex section of the three-dimensional structure main body, and the coating film that includes a titanium alkoxide hydrolysis product, and the coating film has a thickness of 10 nm to 200 nm, wherein
no cracks or peelings of the coating film can be recognized when the concave section and/or the convex section on the surface of the three-dimensional structure is observed with a scanning electron microscope at a magnification of 300; and
the coating film has bioactivity when evaluated under conditions specified in ISO23317.

2. The three-dimensional structure having bioactivity according to claim 1, wherein the coating film has an adhesion strength measured by a 180 degree peeling test in accordance with JIS K 6854 of 40 N/10 mm or higher.

3. The three-dimensional structure having bioactivity according to claim 1 or 2, wherein the coating film has a thickness of 20 nm to 200 nm.

4. The three-dimensional structure having bioactivity according to any one of claims 1 to 3, wherein the three-dimensional structure main body is made of polyetheretherketone.

5. The three-dimensional structure having bioactivity according to any one of claims 1 to 4, which is used as a bone repair material, a joint prosthetic material, or an interbody cage.

6. A method for producing a three-dimensional structure having bioactivity, comprising:
a main body preparation step of preparing a three-dimensional structure main body having a concave section and/or a convex section on a surface;
a dispersion liquid preparation step of preparing a coating film precursor dispersion liquid comprises a coating film precursor that includes a titanium alkoxide partial hydrolysis product by mixing water and 0.08 parts by mole to 1.5 parts by mole of a titanium alkoxide with respect to 37 parts by mole of an organic solvent;
a precursor coating step of coating the coating film precursor dispersion liquid on the surface of the three-dimensional structure main body, and a coating film forming step of forming a coating film that includes the titanium alkoxide hydrolysis product on the surface of the three-dimensional structure main body by rotating the three-dimensional structure main body so that a centrifugal force acts on a center of gravity thereof; and
a bioactivation treatment step of performing bioactivation treatment of the coating film.

7. The method for producing a three-dimensional structure having bioactivity according to claim 6, including a main body modification treatment step of modifying the surface of the three-dimensional structure main body before performing the precursor coating step.

8. The method for producing a three-dimensional structure having bioactivity according to claim 6 or 7, wherein a relative centrifugal acceleration of the centrifugal force acting on the center of gravity of the three-dimensional structure main body is 10 G to 500 G in the coating film forming step.

9. The method for producing a three-dimensional structure having bioactivity according to any one of claims 6 to 8, wherein the three-dimensional structure main body coated with the coating film by the rotation is subjected to drying at a temperature of 50°C to 200°C in the coating film forming step.
